(19) Europäisches Patentamt
European Patent Office
Office européen des brevets

(11) Publication number: **0 273 309 B1**

(12) # EUROPEAN PATENT SPECIFICATION

(45) Date of publication of patent specification: **11.01.95**

(51) Int. Cl.[6]: **A61K 31/41**, A61K 31/44, A61K 31/47

(21) Application number: **87118727.4**

(22) Date of filing: **17.12.87**

(54) **5-Aryl-3H-1,2,4-triazol-3-ones and their use in the treatment of neurodegenerative disorders.**

(30) Priority: **19.12.86 US 944634**
**16.10.87 US 107001**

(43) Date of publication of application:
**06.07.88 Bulletin 88/27**

(45) Publication of the grant of the patent:
**11.01.95 Bulletin 95/02**

(84) Designated Contracting States:
**AT BE CH DE ES FR GB GR IT LI LU NL SE**

(56) References cited:
**EP-A- 0 221 485**
**BE-A- 621 842**
**DD-A- 153 953**
**DD-A- 160 447**
**DE-A- 1 126 882**
**US-A- 3 514 466**

**Synthesis, vol. 10, Oct. 1987, pp. 912-914, J.M. Kane: "The bis(tricyclohexylstannyl) sulfide thionation of 3H-1,2,4-triazol-3-ones"**

**J. of Pharmac. Sciences, vol. 66, no. 7, July 1977, pp. 971-997 S.S. Parmar et al.: "Anticonvulsant activity and selective inhibition of NAD-dependent oxidations in rat brain homogenates by newer mercaptotriazoles"**

(73) Proprietor: **MERRELL DOW PHARMACEUTICALS INC.**
**P.O. Box 156300**
**2110 East Galbraith Road**
**Cincinnati**
**Ohio 45215-6300 (US)**

(72) Inventor: **Miller, Francis P.**
**336 Broadway**
**Loveland**
**Ohio 45140 (US)**
Inventor: **Kane, John M.**
**6813 Dearwester Drive**
**Cincinnati**
**Ohio 45236 (US)**
Inventor: **Sorensen, Stephen**
**3305 Morrison**
**Cincinnati**
**Ohio 45220 (US)**

(74) Representative: **Sgarbi, Renato et al**
**GRUPPO LEPETIT S.p.A.**
**Patent and Trademark Department**
**Via Roberto Lepetit, 34**
**I-21040 Gerenzano (Varese) (IT)**

EP 0 273 309 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Research Communications in Chemical Pathology and , vol. 37, no. 3, Sept. 1982, pp. 499-502, R.K. Jaiswal et al.: "Anticonvulsant activity and monoamine oxidase inhibitory properties of substituted 1,2,4-triazoles"**

**J. of Medicinal Chemistry, vol. 14, no. 3, 1971, pp. 260-262, M.Y. Mhasalkar et al.: "Further studies in substituted 4H-1,2,4-triazoles for possible hypoglycemic activity"**

## Description

This invention relates to the use of 5-aryl-3H-1,2,4-triazol-3-ones, for preparing medicaments for the treatment of neurodegenerative disorders such as tremor and spasticity, cerebral ischemia and stroke.

M.Y. Mhasalkar, et al., (J. Med. Chem. 14, 260-262, 1971) teach that a compound of formula I has hypoglycemic activity. U.S. 3,514,466; BE 621,842 and DE 1,126,882 teach that various compounds of formula I have sedative and hypnotic activity. ZA 65/1537 and NL 6504121 teach that some compounds of formula I have anti-inflammatory, antipyretic and analgetic activity. DD 160,447; DD 153,953 and BE 894,856 teach that some compounds of formula I are herbicides. U.S. 4,414,221 teaches that some compounds of formula I are insecticides and acaricides. JP 50-63119 teaches that some compounds of formula I have anticoccidial activity. None of the uses in the references suggest the use of compounds of formula I for treating seizures or neurodegenerative disorders.

More specifically this invention relates to compounds of the formula:

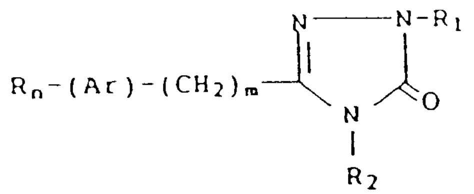

I

wherein

| | |
|---|---|
| Ar | represents phenyl, naphthyl, or an aromatic heterocyclic moiety selected from 2-, 3- or 4-pyridyl, 2- or 3-furyl, 2- or 3-thienyl, 2- or 3-pyrrolyl, N-($C_{1-6}$ alkyl)-pyrrolyl, 6-isoquinolyl, 6-quinolyl and 3-quinolyl, |
| $R_1$ | is hydrogen or $C_{1-6}$ alkyl, |
| $R_2$ | is $C_{1-6}$ alkyl, |
| R | is $C_{1-6}$ alkyl, $C_{1-6}$ alkoxy, hydroxy, halogeno, or trifluoromethyl, and |
| n | is zero, 1 or 2, or |
| $R_n$-(Ar) | is methylenedioxyphenyl, and |
| m | is zero, 1 or 2, |

and the pharmaceutically acceptable salts of the compounds wherein Ar is a nitrogen-containing heterocyclic moiety, for preparing a medicament for treating neurodegenerative disorders.

For R, halogeno preferably represents chloro or fluoro, and methyl and ethyl represent the preferred $C_{1-6}$ alkyl moieties, although all the straight, branched and cyclic manifestations thereof such as n-propyl, cyclopentyl, cyclohexyl and cyclopropyl are herein included. Lower alkoxy radicals include ethers having alkyl moieties paralleling the $C_{1-6}$ alkyl group.

$R_1$ and $R_2$ are preferably methyl or ethyl, although any straight or branched $C_{1-6}$ alkyl group may be used. Compounds wherein $R_1$ is hydrogen are also preferred.

When "Ar" is phenyl, preferably n is one, representing a mono-substituted phenyl moiety with the R-substitutent being a group located at any of the ortho, meta or para positions, although the ortho- and para-substituted compounds are preferred. When Ar is disubstituted (i.e., n is 2), the 2,3-; 2,4-; 2,5-; 2,6-; 3,4-; and 3,5- positions are contemplated. The tautomeric forms are included for each of the compounds embraced within formula I. Except when Ar is phenyl,it is preferred that m is zero. When Ar is phenyl it is preferred that m is zero or one.

When "Ar" of formula I represents a heterocyclic moiety such heterocyclic moieties as 2-, 3- or 4-pyridyl, 2- or 3-furyl, 2- or 3-thienyl, 2- or 3-pyrrolyl, N-($C_{1-6}$ alkyl)-pyrrolyl, 6-isoquinolyl, 6-quinolyl and 3-quinolyl are contemplated. State of the art salts formed with nitrogen-containing heterocyclic moieties are generally employed, with the hydrochloride being one of convenience and general applicability; such salts being formed by standard techniques well known in the art.

When "Ar" represents naphthyl, the preferred isomer is 2-naphthyl with the R moiety being attached thereto at any of the available positions, although positions 5-, 6-, 7-or 8- are preferred for either the mono- or di-R-substituted naphthyl compounds of formula I.

The compounds of Formula I may readily be prepared using processes and techniques analogously known in the art, for example in the method of S. Kuboda and M. Uda, Chem. Pharm. Bull. 21, 1342 (1979), as seen by the following reaction scheme:

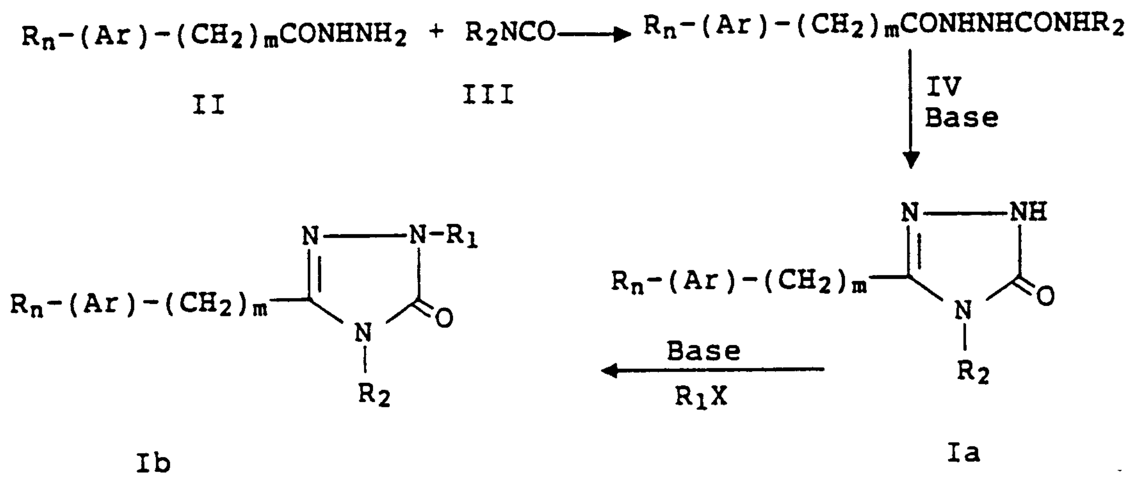


wherein $R_n$-(Ar)-($CH_2$)m, n, $R_1$ and $R_2$ are as defined in formula I, and X is a suitable leaving group.

The preparation of the 1-aroylsemicarbazides (IV) is readily effected by reacting an aroyl hydrazide (II) with an $R_2$-substituted isocyanate (III) by contacting the reactants together in a suitable aprotic solvent, preferably one in which the hydrazide reactant is soluble, e.g., tetrahydrofuran (THF), $CHCl_3$, $CH_2Cl_2$, benzene, toluene, $Et_2O$ and the like. The reaction is quite rapid and may be carried out at O°C to about room temperature and, although the reaction proceeds rapidly, the mixture may be left for 24 hours without any significant decrease in yield. The required hydrazides and isocyanates are readily available but may be prepared by known techniques quite obvious to one of ordinary skill in the art.

The desired 5-aryl-2,4-dihydro-3H-1,2,4-triazol-3-ones (Ia) may be prepared by reacting the semicarbazides (IV) with a base, preferably an aqueous alkali metal hydroxide (e.g., NaOH, KOH) at about 50-120°C, although reflux temperatures are preferred. Normal reaction time is about 7 hours, although 4-24 hours may be needed depending on the temperature of the mixture.

The desired 2,4-disubstituted-2,4-dihydro-3H-1,2,4-triazol-3-ones (Ib) may be prepared by reacting the 4-substituted-2,4-dihydro-3H-1,2,4-triazol-3-ones (Ia) with an appropriate $R_1X$ reactant wherein X is a suitable leaving group, e.g., Cl, Br, $OSO_2CF_3$ and the like. Preferably the reaction takes place in a solution of an aqueous alkali metal hydroxide, (e.g., KOH, NaOH) although more reactive bases (e.g., NaH, KH, LDA) may be used if the reaction is affected under aprotic dry conditions. The reaction preferably takes place at room temperature over periods of about 18 hours to two weeks.

The following specific examples are given to illustrate the preparation of the compounds of this invention although the scope of compounds exemplified is not meant to be limiting, this being so in view of the ease by which the compounds of formula I may be prepared.

## Preparation of Intermediate 1-Aroyl-4-substituted Semicarbazides

### EXAMPLE 1

### 1-(4-Chlorobenzoyl)-4-ethylsemicarbazide

A stirred suspension of 4-chlorobenzoic acid hydrazide (17.1 g, 1.00 x $10^{-1}$ mole), and THF (425 ml) was warmed until homogeneous, at which time ethyl isocyanate (8.7 ml, 1.1 x $10^{-1}$ mole) was added via syringe. A precipitate soon formed. After stirring overnight the reaction mixture was diluted with $Et_2O$ and the precipitate was collected by filtration affording a colorless powder: 23.7 g (98%). Crystallization from ethanol gave a colorless solid: 21.4 g (88%), Mp 237-239°C.

### EXAMPLE 2

### 1-(4-Pyridoyl)-4-methylsemicarbazide

When, in the procedure of Example 1, isonicotinic acid hydrazide is substituted for 4-chlorobenzoic acid hydrazide and methyl isocyanate is substituted for ethyl isocyanate, the title compound is obtained.

EXAMPLE 3

1-(2-Thienoyl)-4-methylsemicarbazide

When in the procedure of Example 1, 2-thiophene-carboxylic acid hydrazide is substituted for 4-chlorobenzoic acid hydrazide and methyl isocyanate is substituted for ethyl isocyanate, the title compound is obtained.

Preparation of 5-Aryl-4-substituted-2,4-dihydro-3H-1,2,4-triazol-3-ones

EXAMPLE 4

5-(4-Chlorophenyl)-2,4-dihydro-4-ethyl-3H-1,2,4-triazol-3-one

1-(4-chlorobenzoyl)-4-ethylsemicarbazide (23.7 g, 9.81 x $10^{-2}$ mole) and 1 molar aqueous NaOH (118 ml, 1.18 x $10^{-1}$ mole) were stirred and warmed to reflux. After refluxing 23 hours, heating was discontinued and the reaction mixture was acidified by the dropwise addition of 1 molar aqueous hydrochloric acid (130 ml, 1.30 x $10^{-1}$ mole). A colorless solid formed as the reaction mixture was acidified and, after cooling in an ice bath, this was collected by filtration. Crystallization from isopropanol gave colorless spars: 18.2 g (83%), Mp 188-189 °C.

EXAMPLE 5

2,4-Dihydro-4-methyl-5-(4-pyridinyl)-3H-1,2,4-triazol-3-one

When, in the procedure of Example 4, 1-(4-pyridoyl)-4-methylsemicarbazide is substituted for 1-(4-chlorobenzoyl)-4-ethylsemicarbazide, the title compound is obtained. Mp 249-251 °C.

EXAMPLE 6

5-(2-Thienyl)-2,4-dihydro-4-methyl-3H-1,2,4-triazol-3-one

When, in the procedure of Example 4, 1-(2-thienoyl)-4-methylsemicarbazide is substituted for 1-(4-chlorobenzoyl)-4-ethylsemicarbazide, the title compound is obtained. Mp 183-185 °C.

Preparation of 5-Aryl-2,4-dihydro-2,4-disubstituted-3H-1,2,4-triazol-3-ones

EXAMPLE 7

5-(4-Chlorophenyl)-2,4-dihydro-4-ethyl-2-methyl-3H-1,2,4-triazol-3-one

To a stirred, room temperature solution of 5-(4-chlorophenyl)-2,4-dihydro-4-ethyl-3H-1,2,4-triazol-3-one (6.00 g, 2.68 x $10^{-2}$ mole) and 1 molar aqueous NaOH (30.0 ml, 3.00 x $10^{-2}$ mole) was added a solution of methyl iodide (2.5 ml, 4.0 x $10^{-2}$ mole) and ethanol (10 ml). After stirring overnight at room temperature, the reaction mixture was transferred to a separatory funnel where it was extracted three times with EtOAc. The EtOAc extracts were combined, washed with saturated aqueous NaCl, and dried over $Na_2SO_4$. The drying agent was removed by filtration and the filtrate was evaporated at reduced pressure leaving an oil which slowly solidified. Chromatography and crystallization from cyclohexane gave small colorless needles: 3.4 g (53%), Mp 73-75 °C.

EXAMPLE 8

5-(2-Thienyl)-2,4-dihydro-2,4-dimethyl-3H-1,2,4-triazol-3-one

When, in the procedure of Example 7, 5-(2-thienyl)-2,4-dihydro-4-methyl-3H-1,2,4-triazol-3-one is substituted for 5-(4-chlorophenyl)-2,4-dihydro-4-ethyl-3H-1,2,4-triazol-3-one, the title compound is obtained. Mp 108-110 °C.

In a similar manner the following compounds also may be prepared.

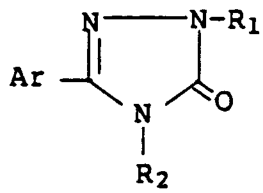

| Ar | $R_1$ | $R_2$ | mp (°C) |
|---|---|---|---|
| $C_6H_5$ | H | $CH_3$ | 177-178 |
| $C_6H_5$ | $CH_3$ | $CH_3$ | 140-141 |
| $C_6H_5$ | $C_2H_5$ | $CH_3$ | 87-89 |
| $C_6H_5$ | H | $C_2H_5$ | 163-165 |
| $C_6H_5$ | $CH_3$ | $C_2H_5$ | oil |
| $2-ClC_6H_4$ | H | $CH_3$ | 168-170 |
| $2-ClC_6H_4$ | $CH_3$ | $CH_3$ | 61-63 |
| $4-ClC_6H_4$ | H | $CH_3$ | 213-215 |
| $4-ClC_6H_4$ | $CH_3$ | $CH_3$ | 126-128 |
| $4-ClC_6H_4$ | $C_2H_5$ | $CH_3$ | 79-81 |
| $4-ClC_6H_4$ | $C_2H_5$ | $C_2H_5$ | 62-64 |
| $4-ClC_6H_4$ | $n-C_3H_7$ | $C_2H_5$ | oil |
| $2-FC_6H_4$ | H | $CH_3$ | 189-191 |
| $2-FC_6H_4$ | $CH_3$ | $CH_3$ | 69-71 |
| $4-FC_6H_4$ | H | $CH_3$ | 216-218 |
| $4-FC_6H_4$ | $CH_3$ | $CH_3$ | 104-106 |
| $3,4-Cl_2C_6H_3$ | H | $CH_3$ | 170-172 |
| $3,4-Cl_2C_6H_3$ | $CH_3$ | $CH_3$ | 107-109 |
| $4-CH_3C_6H_4$ | H | $CH_3$ | 206-208 |
| $4-CH_3C_6H_4$ | $CH_3$ | $CH_3$ | 92-94 |
| $4-CH_3O-3-(n-C_4H_9O)C_6H_3$ | H | $CH_3$ | 96-98 |
| $4-CH_3O-3-(n-C_4H_9O)C_6H_3$ | $CH_3$ | $CH_3$ | 112-114 |
| $4-CH_3O-3-(cyclo-C_5H_9O)C_6H_3$ | H | $CH_3$ | 184-186 |
| $4-CH_3O-3-(cyclo-C_5H_9O)C_6H_3$ | $CH_3$ | $CH_3$ | 153-155 |

Triazol-3-ones of formula I have been shown to antagonize the seizures induced in mice by quinolinic acid, a compound thought to be an agonist at the N-methyl-D-aspartate (NMDA)-subtype of the glutamate receptor. In addition, triazol-3-ones of formula I have been shown to antagonize hippocampal neurodegeneration after carotid artery occlusion in gerbils. These results indicate that the active triazol-3-ones will have therapeutic utility in the treatment of neurodegenerative disorders such as stroke, cerebral ischemia, Huntington's disease, parkinsonism and Alzheimer's disease. Antagonists of excitatory amino acids have been found to have potential applications in the treatment of neurodegenerative disorders. Such

disorders include tremor and spasticity as shown in drug-induced parkinsonism, stroke, cerebral ischemia and Alzheimer's disease.

The compounds of this invention will exert activity useful in the treatment of neurodegenerative disorders at oral dosage levels of about 0.25 to 40 mg/kg of body weight per day. Of course the degree of severity of the disease, age of the patient and other factors normally considered by the attending diagnostician will influence the individual regimen for each patient. In general, the parenterally administered doses are about 1/4 to 1/2 that of the orally administered dose.

For oral administration the compounds can be formulated into solid or liquid preparations such as capsules, pills, tablets, troches, powders, solutions, suspensions or emulsions. The solid unit dosage forms can be a capsule which can be of the ordinary gelatin type containing, for example, lubricants and inert fillers such as lactose, sucrose or cornstarch. In another embodiment the compounds of general formula I can be tableted with conventional tablet bases such as lactose, sucrose and cornstarch, in combination with binders such as acacia, cornstarch or gelatin, disintegrating agents such as potato starch or alginic acid, and a lubricant such as stearic acid or magnesium stearate.

For parenteral administration, the compounds may be administered as injectable dosages of a solution or suspension of the compound in a physiologically acceptable diluent with a pharmaceutical carrier which can be a sterile liquid such as water, alcohol, oils and other acceptable organic solvents, with or without the addition of a surfactant and other pharmaceutically acceptable adjuvants. Illustrative of oils which can be employed in these preparations are those of petroleum, animal, vegetable or synthetic origin, for example, peanut oil, soybean oil and mineral oil. In general, water, saline, aqueous dextrose and related sugar solutions, ethanol, glycols such as propylene glycol or polyethylene glycol, or 2-pyrrolidone are preferred liquid carriers, particularly for injectable solutions.

The compounds can be administered in the form of a depot injection or implant preparation which may be formulated in such a manner as to permit a sustained release of the active ingredient. The active ingredient can be compressed into pellets or small cylinders and implanted subcutaneously or intramuscularly as depot injections or implants. Implants may employ inert materials such as biodegradable polymers or synthetic silicones, for example, Silastic®, a silicone rubber manufactured by the Dow-Corning Corporation.

As is true for most classes of compounds generally suitable as therapeutic agents, certain subgeneric groups and specific members of that class, in the light of their overall biological profile, are preferred. In this instance those compounds wherein m is zero are preferred, with those wherein m is one being next preferred. The preferred Ar moiety is phenyl. The preferred R substituent is chloro, with the chloro being at the 2- or 4-positions of the aromatic moiety being preferred. It is preferred that the alkyl substituent at R positions be methyl and ethyl, with hydrogen, methyl or ethyl being the preferred groups for $R_1$. Particularly preferred compounds are

5-(4-chlorophenyl)-2,4-dihydro-4-ethyl-2-methyl-3H-1,2,4-triazol-3-one,

5-(4-chlorophenyl)-2,4-dihydro-4-ethyl-3H-1,2,4-triazol-3-one, and

5-(2-chlorophenyl)-2,4-dihydro-4-methyl-3H-1,2,4-triazol-3-one.

In the present description and claims, the term therapeutic "treatment" includes any kind of treatment such as prophylaxis and cure.

## Claims

**1.** Use of a compound of the formula

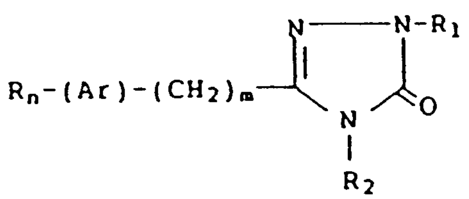

I

wherein

Ar represents phenyl, naphthyl, or an aromatic heterocyclic moiety selected from 2-, 3- or 4-pyridyl, 2- or 3-furyl, 2- or 3-thienyl, 2- or 3-pyrrolyl, N-($C_{1-6}$ alkyl)-pyrrolyl, 6-isoquinolyl, 6-quinolyl and 3-quinolyl,

| | |
|---|---|
| $R_1$ | is hydrogen or $C_{1-6}$ alkyl, |
| $R_2$ | is $C_{1-6}$ alkyl, |
| R | is $C_{1-6}$ alkyl, $C_{1-6}$ alkoxy, hydroxy, halogeno, or trifluoromethyl, and |
| n | is zero, 1 or 2, or |
| $R_n$-(Ar) | is methylenedioxyphenyl, and |
| m | is zero, 1 or 2, |

and the pharmaceutically acceptable salts of the compounds wherein Ar is a nitrogen-containing heterocyclic moiety, for preparing a medicament for treating neurodegenerative disorders.

**2.** Use of a compound of claim 1 wherein Ar in said compound represents phenyl.

**3.** Use of a compound of claim 1 or 2 wherein m in said compound represents zero.

**4.** Use of a compound of claim 1, 2 or 3 wherein $R_1$ in said compound represents hydrogen, methyl or ethyl.

**5.** Use of a compound of claim 1, 2 or 3 wherein $R_2$ in said compound represents methyl or ethyl.

**6.** Use of a compound of claim 1 wherein Ar in said compound is phenyl, m is zero and R is halogeno.

**7.** Use of a compound of claim 6 wherein R in said compound is chloro.

**8.** Use of a compound of claim 1 wherein $R_n$-(Ar)-$(CH_2)_m$ in said compound represents 4-chlorophenyl, $R_1$ is methyl and $R_2$ is ethyl.

**9.** Use of a compound of claim 1 wherein $R_n$-(Ar)-$(CH_2)_m$ in said compound represents 4-chlorophenyl, $R_1$ is hydrogen and $R_2$ is ethyl.

**10.** Use of a compound of claim 1 wherein $R_n$-(Ar)-$(CH_2)_m$ in said compound represents 2-chlorophenyl, $R_1$ is hydrogen and $R_2$ is methyl.

**11.** Use according to any one of claims 1 to 10 for preparing a medicament for the treatment of stroke.

**12.** Use according to any one of claims 1 to 10 for preparing a medicament for the treatment of cerebral ischemia.

**13.** Use according to any one of claims 1 to 10 for preparing a medicament for the treatment of parkinsonism.

**14.** Use according to any one of claims 1 to 10 for preparing a medicament for the treatment of Alzheimer's disease.

**15.** Use according to any one of claims 1 to 10 for preparing a medicament for the treatment of Huntington's disease.

**Patentansprüche**

**1.** Verwendung einer Verbindung der Formel

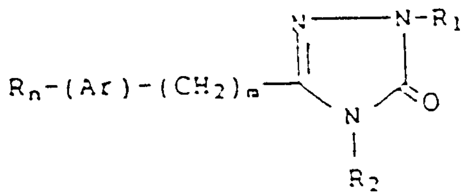

(I)

in der

| | |
|---|---|
| Ar | eine Phenyl- oder Naphthylgruppe oder einen aromatischen heterocyclischen Rest darstellt, der aus einer 2-, 3- oder 4-Pyridyl-, 2- oder 3-Furyl-, 2- oder 3-Thienyl-, 2- oder 3-Pyrrolyl-, N-($C_{1-6}$-Alkyl)-pyrrolyl-, 6-Isochinolyl-, 6-Chinolyl- und 3-Chinolylgruppe ausgewählt ist, |
| $R_1$ | ein Wasserstoffatom oder einen $C_{1-6}$-Alkylrest bedeutet, |
| $R_2$ | einen $C_{1-6}$-Alkylrest bedeutet, |
| R | einen $C_{1-6}$-Alkyl-, $C_{1-6}$-Alkoxy-, Hydroxy-, Halogen- oder Trifluormethylrest bedeutet, und |
| n | 0, 1 oder 2 ist, oder |
| $R_n$-(Ar) | eine Methylendioxyphenylgruppe bedeutet, und |
| m | 0, 1 oder 2 ist, |

und der pharmazeutisch verträglichen Salze der Verbindungen, in denen Ar einen Stickstoff enthaltenden heterocyclischen Rest bedeutet, zur Herstellung eines Arzneimittels zur Behandlung von neurodegenerativen Beschwerden.

**2.** Verwendung einer Verbindung nach Anspruch 1, in der Ar in der Verbindung eine Phenylgruppe bedeutet.

**3.** Verwendung einer Verbindung nach Anspruch 1 oder 2, in der m in der Verbindung 0 ist.

**4.** Verwendung einer Verbindung nach Anspruch 1, 2 oder 3, in der $R_1$ in der Verbindung ein Wasserstoffatom, eine Methyl- oder Ethylgruppe bedeutet.

**5.** Verwendung einer Verbindung nach Anspruch 1, 2 oder 3, in der $R_2$ in der Verbindung eine Methyl- oder Ethylgruppe bedeutet.

**6.** Verwendung einer Verbindung nach Anspruch 1, in der Ar in der Verbindung eine Phenylgruppe bedeutet, m 0 ist und R ein Halogenatom darstellt.

**7.** Verwendung einer Verbindung nach Anspruch 6, in der R in der Verbindung ein Chloratom bedeutet.

**8.** Verwendung einer Verbindung nach Anspruch 1, in der der Rest $R_n$-(Ar)-$(CH_2)_m$ in der Verbindung eine 4-Chlorphenylgruppe darstellt, $R_1$ eine Methylgruppe bedeutet und $R_2$ eine Ethylgruppe ist.

**9.** Verwendung einer Verbindung nach Anspruch 1, in der der Rest $R_n$-(Ar)-$(CH_2)_m$ in der Verbindung eine 4-Chlorphenylgruppe darstellt, $R_1$ ein Wasserstoffatom bedeutet und $R_2$ eine Ethylgruppe ist.

**10.** Verwendung einer Verbindung nach Anspruch 1, in der der Rest $R_n$-(Ar)-$(CH_2)_m$ in der Verbindung eine 2-Chlorphenylgruppe darstellt, $R_1$ ein Wasserstoffatom bedeutet und $R_2$ eine Methylgruppe ist.

**11.** Verwendung gemäß einem der Ansprüche 1 bis 10 zur Herstellung eines Medikaments zur Behandlung von Schlaganfällen.

**12.** Verwendung gemäß einem der Ansprüche 1 bis 10 zur Herstellung eines Arzneimittels zur Behandlung von zerebraler Ischämie.

**13.** Verwendung gemäß einem der Ansprüche 1 bis 10 zur Herstellung eines Arzneimittels zur Behandlung der Parkinson'schen Krankheit.

**14.** Verwendung gemäß einem der Ansprüche 1 bis 10 zur Herstellung eines Arzneimittels zur Behandlung der Alzheimer'schen Krankheit.

**15.** Verwendung nach einem der Ansprüche 1 bis 10 zur Herstellung eines Arzneimittels zur Behandlung der Huntington'schen Krankheit.

## Revendications

**1.** Utilisation d'un composé de formule

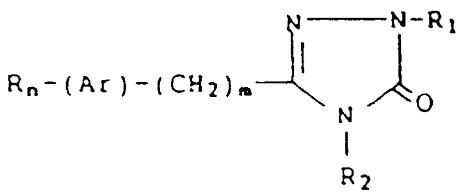

I

dans laquelle :

Ar est phényle, naphtyle ou un reste hétérocyclique aromatique choisi parmi 2-, 3- ou 4-pyridyle, 2- ou 3-furyle, 2- ou 3-thiényle, 2- ou 3-pyrrolyle, N-(alkyl en $C_1$-$C_6$)pyrrolyle, 6-isoquinolyle, 6-quinolyle et 3-quinolyle,

$R_1$ est hydrogène ou alkyle en $C_1$-$C_6$,

$R_2$ est alkyle en $C_1$-$C_6$,

R est alkyle en $C_1$-$C_6$, alcoxy en $C_1$-$C_6$, hydroxy, halogéno ou trifluorométhyle, et

n est 0, 1 ou 2, ou

$R_n$-(Ar) est méthylènedioxyphényle, et

m est 0, 1 ou 2,

et des sels pharmaceutiquement acceptables des composés dans lesquels Ar est un reste hétérocyclique azoté, pour la préparation d'un médicament pour le traitement de troubles neurodégénératifs.

**2.** Utilisation d'un composé selon la revendication 1, dans laquelle, dans ledit composé, Ar représente un phényle.

**3.** Utilisation d'un composé selon la revendication 1 ou 2, dans laquelle, dans ledit composé, m représente zéro.

**4.** Utilisation d'un composé selon la revendication 1, 2 ou 3, dans laquelle, dans ledit composé, $R_1$ représente l'hydrogène, un méthyle ou un éthyle;

**5.** Utilisation d'un composé selon la revendication 1, 2 ou 3, dans laquelle, dans ledit composé, $R_2$ représente un méthyle ou un éthyle.

**6.** Utilisation d'un composé selon la revendication 1, dans laquelle, dans ledit composé, Ar est phényle, m est zéro et R est halogéno.

**7.** Utilisation d'un composé selon la revendication 6, dans laquelle, dans ledit composé, R est chloro.

**8.** Utilisation d'un composé selon la revendication 1, dans laquelle, dans ledit composé, $R_n$-(Ar)-$(CH_2)_m$ représente un 4-chlorophényle, $R_1$ est méthyle et $R_2$ est éthyle.

**9.** Utilisation d'un composé selon la revendication 1, dans laquelle, dans ledit composé, $R_n$-(Ar)-$(CH_2)_m$ représente un 4-chlorophényle, $R_1$ est hydrogène et $R_2$ est éthyle.

**10.** Utilisation d'un composé selon la revendication 1, dans laquelle, dans ledit composé, $R_n$-(Ar)-$(CH_2)_m$ représente un 2-chlorophényle, $R_1$ est hydrogène et $R_2$ est méthyle.

**11.** Utilisation selon l'une quelconque des revendications 1 à 10 pour la préparation d'un médicament pour le traitement des attaques.

**12.** Utilisation selon l'une quelconque des revendications 1 à 10 pour la préparation d'un médicament pour le traitement de l'ischémie cérébrale.

**13.** Utilisation selon l'une quelconque des revendications 1 à 10 pour la préparation d'un médicament pour le traitement du parkinsonisme.

**14.** Utilisation selon l'une quelconque des revendications 1 à 10 pour la préparation d'un médicament pour le traitement de la maladie d'Alzheimer.

**15.** Utilisation selon l'une quelconque des revendications 1 à 10 pour la préparation d'un médicament pour le traitement de la chorée de Huntington.